(19) **Europäisches Patentamt European Patent Office Office européen des brevets**

(11) **EP 4 597 174 A1**

(12) **EUROPEAN PATENT APPLICATION**
published in accordance with Art. 153(4) EPC

(43) Date of publication:
06.08.2025  Bulletin 2025/32

(21) Application number: 22962577.7

(22) Date of filing: 09.12.2022

(51) International Patent Classification (IPC):
*G01V 11/00* (2006.01)

(52) Cooperative Patent Classification (CPC):
G01V 11/00; Y02E 30/30

(86) International application number:
PCT/CN2022/138085

(87) International publication number:
WO 2024/082401 (25.04.2024 Gazette 2024/17)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 17.10.2022  CN 202211271154

(71) Applicant: China National Petroleum Corporation
Beijing 100007 (CN)

(72) Inventors:
• WU, Yiping
  Beijing 100083 (CN)
• DOU, Lirong
  Beijing 100000 (CN)
• WU, Xiaozhi
  Beijing 100083 (CN)
• TAO, Shizhen
  Beijing 100083 (CN)
• WANG, Jianjun
  Beijing 100083 (CN)
• WANG, Qing
  Beijing 100083 (CN)
• LI, Qian
  Beijing 100083 (CN)
• HUANG, Fei
  Beijing 100083 (CN)

(74) Representative: Ström & Gulliksson AB
P.O. Box 4188
203 13 Malmö (SE)

(54) **HELIUM GENESIS RESOURCE EVALUATION METHOD AND APPARATUS**

(57)    A helium genesis resource evaluation method and apparatus, a processor, and a storage medium, relating to the technical field of helium genesis resource evaluation. The method comprises: determining the content of a decayed daughter isotope in a helium source rock at a target area; determining the amount of substance of helium in the helium source rock on the basis of the content of the decayed daughter isotope in the helium source rock; determining a total helium production amount of the helium source rock on the basis of the amount of substance of the helium in the helium source rock; determining a migration-accumulation coefficient of the helium in the helium source rock at the target area; and determining a helium accumulation resource amount on the basis of the migration-accumulation coefficient and the total helium production amount of the helium source rock. The method can realize effective evaluation on helium resources, and has an important guidance effect on conducting a research on helium reservoir formation and enrichment rule.

EP 4 597 174 A1

Acquiring an absolute age of a helium source rock, a density of the helium source rock, the content of a radioactive element in the helium source rock, and a decay constant of the radioactive element in the helium source rock at a target area /S110

Determining a volume of the helium source rock at the target area /S120

Determining the content of an undecayed parent isotope in the helium source rock on the basis of the density of the helium source rock, the content of the radioactive element in the helium source rock and the volume of the helium source rock /S130

Determining the content of a decayed daughter isotope in the helium source rock on the basis of the absolute age of the helium source rock, the decay constant of the radioactive element in the helium source rock and the content of the undecayed parent isotope in the helium source rock /S140

Determining the amount of substance of helium in the helium source rock on the basis of the content of the decayed daughter isotope in the helium source rock /S150

Determining a total helium production amount of the helium source rock on the basis of the amount of substance of the helium in the helium source rock /S160

Determining a migration-accumulation coefficient of the helium in the helium source rock at the target area /S170

Determining a helium accumulation resource amount on the basis of the migration-accumulation coefficient and the total helium production amount of the helium source rock /S180

FIG. 2

## Description

### Field of the Invention

The present application relates to the technical field of helium resource evaluation, in particular to a helium genesis resource evaluation method, a helium genesis resource evaluation apparatus, a machine-readable storage medium and a processor.

### Background of the Invention

Helium is referred to as "golden gas". The inventors have found that due to the difficulty in determining the thickness of a helium source rock, a migration-accumulation coefficient, and a mantle source rock, there is currently no mature helium production method at home and abroad to carry out helium resource evaluation. The inventors have found that although a helium percentage method is accurate in calculation, it relies on the quantity and quality of helium data points, and the accuracy of natural gas reserves. Also, when the resource amount is calculated by a genesis method, the calculation error of the resource amount is large due to the difficulty in determining the thickness of the helium source rock, the migration-accumulation coefficient, and the mantle source rock. The inventors have also found that although helium and natural gas accumulate in the same trap, there is no correlation between migration-accumulation coefficients of natural gas and helium due to completely different mechanisms of helium production and hydrocarbon production, and the migration and accumulation of helium reservoirs has its own law.

In view of the above problems, the present application proposes a helium genesis resource evaluation method that solves the above problems or at least partially solves the above problems.

### Summary of the Invention

An object of embodiments of the present application is to provide a helium genesis resource evaluation method, a helium genesis resource evaluation apparatus, a machine-readable storage medium, and a processor. The method can realize efficient evaluation of helium resources, and has an important guidance effect on conducting a research on helium reservoir formation and enrichment rule.

In order to achieve the above object, a first aspect of the present application provides a helium genesis resource evaluation method, including:

acquiring an absolute age of a helium source rock, a density of the helium source rock, the content of a radioactive element in the helium source rock, and a decay constant of the radioactive element in the helium source rock at a target area;
determining a volume of the helium source rock at the target area;
determining the content of an undecayed parent isotope in the helium source rock on the basis of the density of the helium source rock, the content of the radioactive element in the helium source rock and the volume of the helium source rock;
determining the content of a decayed daughter isotope in the helium source rock on the basis of the absolute age of the helium source rock, the decay constant of the radioactive element in the helium source rock and the content of the undecayed parent isotope in the helium source rock;
determining the amount of substance of helium in the helium source rock on the basis of the content of the decayed daughter isotope in the helium source rock;
determining a total helium production amount of the helium source rock on the basis of the amount of substance of the helium in the helium source rock;
determining a migration-accumulation coefficient of the helium in the helium source rock at the target area; and
determining a helium accumulation resource amount on the basis of the migration-accumulation coefficient and the total helium production amount of the helium source rock.

Wherein, determining the volume of the helium source rock at the target area includes:

determining a bottom boundary depth of the helium source rock at the target area based on a preset temperature;
determining a top surface depth of the helium source rock and a distribution area of the helium source rock at the target area;
determining a thickness of the helium source rock based on the bottom boundary depth of the helium source rock and the top surface depth of the helium source rock; and
determining the volume of the helium source rock based on the thickness of the helium source rock and the distribution area of the helium source rock.

In the embodiments of the present application, determining the bottom boundary depth of the helium source rock at the target area includes: determining the bottom boundary depth of the helium source rock at the target area based on the preset temperature.

In the embodiments of the present application, determining the bottom boundary depth of the helium source rock at the target area based on the preset temperature includes:
determining a formation depth corresponding to the preset temperature as the bottom boundary depth of the helium source rock; wherein the helium source rock includes granite and a volcanic rock.

In the embodiments of the present application, the preset temperature ranges from 220°C to 240°C.

In the embodiments of the present application, determining the bottom boundary depth of the helium source rock, the top surface depth of the helium source rock, and the distribution area of the helium source rock at the target area includes:
determining the bottom boundary depth of the helium source rock, the top surface depth of the helium source rock, and the distribution area of the helium source rock at the target area based on seismic data and drilling data; wherein, the helium source rock includes a coal seam and a mud shale.

In the embodiments of the present application, determining the distribution area of the helium source rock based on the seismic data and the drilling data includes:
obtaining the distribution area of the helium source rock by a facet integration method based on the seismic data and the drilling data for a helium source rock having an unstable thickness or a discontinuous distribution.

In the embodiments of the present application, the formation depth corresponding to the preset temperature is determined as the bottom boundary depth of the helium source rock by a formula (1):

$$H_{bottom} = (t_{preset\ temperature} - t_{surface\ temperature})/K + 50 \qquad (1);$$

wherein $H_{bottom}$ represents the bottom boundary depth of the helium source rock in m;
$t_{surface\ temperature}$ represents the surface temperature in °C; and
K represents a geothermal gradient in °C/100 m.

In the embodiments of the present application, the thickness of the helium source rock is determined based on the bottom boundary depth of the helium source rock and the top surface depth of the helium source rock by a formula (2):

$$h = H_{bottom} - H_{top} \qquad (2);$$

wherein h represents the thickness of the helium source rock in m;
$H_{bottom}$ represents the bottom boundary depth of the helium source rock in m; and
$H_{top}$ represents the top surface depth of the helium source rock in m.

In the embodiments of the present application, the volume of the helium source rock is determined based on the thickness of the helium source rock and the distribution area of the helium source rock by a formula (3):

$$V = h \times S \qquad (3);$$

wherein V represents the volume of the helium source rock in $m^3$;
h represents the thickness of the helium source rock in m; and
S represents the distribution area of the helium source rock in $m^2$.

In the embodiments of the present application, the content of the undecayed parent isotope in the helium source rock is determined on the basis of the density of the helium source rock, the content of the radioactive element in the helium source rock and the volume of the helium source rock by a formula (4):

$$P = v \times \rho_{source\ rock} \times x \qquad (4);$$

wherein P represents the content of the undecayed parent isotope in the helium source rock in mol;
v represents the volume of the helium source rock in $m^3$;

$\rho_{\text{source rock}}$ represents the density of the helium source rock in $t/m^3$; and

x represents the content of the radioactive element in the helium source rock in mol/g.

In the embodiments of the present application, the content of the decayed daughter isotope in the helium source rock is determined on the basis of the absolute age of the helium source rock, the decay constant of the radioactive element in the helium source rock and the content of the undecayed parent isotope in the helium source rock by a formula (5):

$$D = (e^{\lambda t}-1) \times v \times \rho_{\text{source rock}} \times x \qquad (5);$$

wherein D represents the content of the decayed daughter isotope in the helium source rock in mol;

$\lambda$ represents a decay constant of an isotope of the radioactive element in the helium source rock, dimensionless;

t represents an absolute geological age of the helium source rock in millions of years;

v represents the volume of the helium source rock in $m^3$;

$\rho_{\text{source rock}}$ represents the density of the helium source rock in $t/m^3$; and

x represents the content of the radioactive element in the helium source rock in mol/g.

In the embodiments of the present application, the radioactive element in the helium source rock includes: 238U and 232Th;

for decay of 238U, the amount of substance of the helium in the helium source rock is determined on the basis of the content of the decayed daughter isotope in the helium source rock by a formula (6):

$$n = D \times 8/238 \qquad (6);$$

for decay of 232Th, the amount of substance of the helium in the helium source rock is determined on the basis of the content of the decayed daughter isotope in the helium source rock by a formula (7):

$$n = D \times 6/232 \qquad (7);$$

wherein n represents the amount of substance of the helium in the helium source rock, and D represents the content of the decayed daughter isotope in the helium source rock.

In the embodiments of the present application, the total helium production amount of the helium source rock is determined on the basis of the amount of substance of the helium in the helium source rock by a formula (8):

$$V_{\text{He production}} = n \times M/\rho_{\text{he}} \qquad (8);$$

wherein $V_{\text{He production}}$ represents the total helium production amount, i.e., a helium production resource amount in $m^3$;

n represents the amount of substance of the helium in the helium source rock;

M represents a molar mass of the total helium production amount; and

$\rho_{\text{he}}$ represents the density of helium and is 0.1786 g/L.

In the embodiments of the present application, the migration-accumulation coefficient of the helium in the helium source rock at the target area is determined by a formula (9):

$$\Phi = \Phi1 \times \Phi2 \qquad (9);$$

wherein $\Phi1$ is a helium discharge rate of the helium source rock in %; and $\Phi2$ is a degassing rate of water-soluble helium in %.

In the embodiments of the present application, the helium accumulation resource amount is determined on the basis of the migration-accumulation coefficient and the total helium production amount of the helium source rock by a formula (10):

$$V_{\text{he accumulation}} = \Phi \times V_{\text{he production}} \qquad (10);$$

wherein $V_{he\ accumulation}$ represents a helium accumulation amount, i.e., the helium accumulation resource amount in $m^3$;

$\Phi$ represents the migration-accumulation coefficient in %; and

$V_{he\ production}$ represents the total helium production amount in $m^3$.

In the embodiments of the present application, the helium includes 3He and 4He, and the method further includes:

determining resource amount of mantle source helium and crust source helium by a ratio of 3He/4He according to a binary composite formula.

A second aspect of the present application provides a helium genesis resource evaluation apparatus, including:

an acquisition module configured to acquire an absolute age of a helium source rock, a density of the helium source rock, the content of a radioactive element in the helium source rock, and a decay constant of the radioactive element in the helium source rock at a target area;

a first determining module configured to determine a volume of the helium source rock at the target area;

a second determining module configured to determine the content of an undecayed parent isotope in the helium source rock on the basis of the density of the helium source rock, the content of the radioactive element in the helium source rock and the volume of the helium source rock;

a third determining module configured to determine the content of a decayed daughter isotope in the helium source rock on the basis of the absolute age of the helium source rock, the decay constant of the radioactive element in the helium source rock and the content of the undecayed parent isotope in the helium source rock;

a fourth determining module configured to determine the amount of substance of helium in the helium source rock on the basis of the content of the decayed daughter isotope in the helium source rock;

a fifth determining module configured to determine a total helium production amount of the helium source rock on the basis of the amount of substance of the helium in the helium source rock;

a sixth determining module configured to determine a migration-accumulation coefficient of the helium in the helium source rock at the target area; and

a seventh determining module configured to determine a helium accumulation resource amount on the basis of the migration-accumulation coefficient and the total helium production amount of the helium source rock.

Wherein, the first determining module includes:

a bottom boundary depth determining module configured to determine a bottom boundary depth of the helium source rock at the target area based on a preset temperature;

a top surface depth and distribution area determining module configured to determine a top surface depth of the helium source rock and a distribution area of the helium source rock at the target area;

a thickness determining module configured to determine a thickness of the helium source rock based on the bottom boundary depth of the helium source rock and the top surface depth of the helium source rock; and

a volume determining module configured to determine the volume of the helium source rock based on the thickness of the helium source rock and the distribution area of the helium source rock.

A third aspect of the present application provides a processor, configured to perform the helium genesis resource evaluation method described above.

A fourth aspect of the present application provides a machine-readable storage medium, having stored thereon instructions, which, when executed by a processor, cause the processor to be configured to perform the helium genesis resource evaluation method described above.

Compared with the prior art, the above technical solutions of the present application have the following beneficial effects:

According to the helium genesis resource evaluation method and apparatus, the storage medium and the processor proposed in the present application, the method determines the amount of substance of the helium in the helium source rock from the content of the decayed daughter isotope in the helium source rock, then determines the total helium production amount of the helium source rock on the basis of the amount of substance of the helium in the helium source rock, and finally determines the helium accumulation resource amount on the basis of the migration-accumulation coefficient of the helium in the helium source rock. The present application can realize efficient evaluation of helium resources through the above method, and has an important guidance effect on conducting a research on helium reservoir formation and enrichment rule.

Other features and advantages of the embodiments of the present application will be described in detail in the subsequent Detailed Description.

**Brief Description of Drawings**

The accompanying drawings are used to provide a further understanding of the embodiments of the present application and constitute a part of this specification, and together with the detailed description below serve to explain, but not to limit, the embodiments of the present application. In the drawings:

FIG. 1 schematically illustrates an application environment of a helium genesis resource evaluation method according to an embodiment of the present application;

FIG. 2 schematically illustrates an overall flow diagram of the helium genesis resource evaluation method according to the embodiment of the present application;

FIG. 3 schematically illustrates a flow chart of calculating a volume of a helium source rock at a target area in the helium genesis resource evaluation method according to the embodiment of the present application;

FIG. 4 schematically illustrates a curve graph of Henry's constants of He, $N_2$ and $CH_4$ as a function of temperature according to an embodiment of the present application;

FIG. 5 schematically illustrates an effective thickness of the helium source rock according to an embodiment of the present application;

FIG. 6 schematically illustrates a helium reservoir formation mode and process according to an embodiment of the present application;

FIG. 7 is a structural block diagram schematically illustrating a helium genesis resource evaluation apparatus according to an embodiment of the present application; and

FIG. 8 schematically illustrates an internal structure of a computer device according to an embodiment of the present application.

**Detailed Description of the Embodiments**

To make the objects, technical solutions and advantages of the embodiments of the present application more clear, the technical solutions in the embodiments of the present application will be clearly and completely described below in conjunction with the accompanying drawings in the embodiments of the present application, and it should be understood that the specific embodiments described herein are only used to illustrate and explain the embodiments of the present application, and are not used to limit the embodiments of the present application. Based on the embodiments in the present application, all other embodiments obtained by those of ordinary skill in the art without making inventive step belong to the scope of protection of the present application.

It should be noted that if there are descriptions involving "first", "second", etc. in the embodiments of the present application, the descriptions of "first", "second", etc. are for descriptive purposes only, and cannot be understood as indicating or implying relative importance thereof or implicitly indicating the number of technical features indicated. Thus, features defined as "first" and "second" may explicitly or implicitly include at least one of the features. In addition, the technical solutions of the embodiments may be combined with each other, but it must be based on that those of ordinary skill in the art can implement the combination of the technical solutions, and when the combination of the technical solutions contradicts each other or cannot be implemented, it should be considered that the combination of the technical solutions does not exist and is not within the scope of protection of the present application.

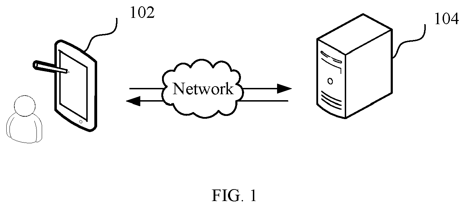

A helium genesis resource evaluation method provided in the present application can be applied to an application environment shown in FIG. 1. A terminal 102 communicates with a server 104 through a network. The terminal 102 may be, but is not limited to, various personal computers, notebook computers, smartphones, tablet computers and portable wearable devices, and the server 104 may be implemented as a stand-alone server or a server cluster composed of a plurality of servers.

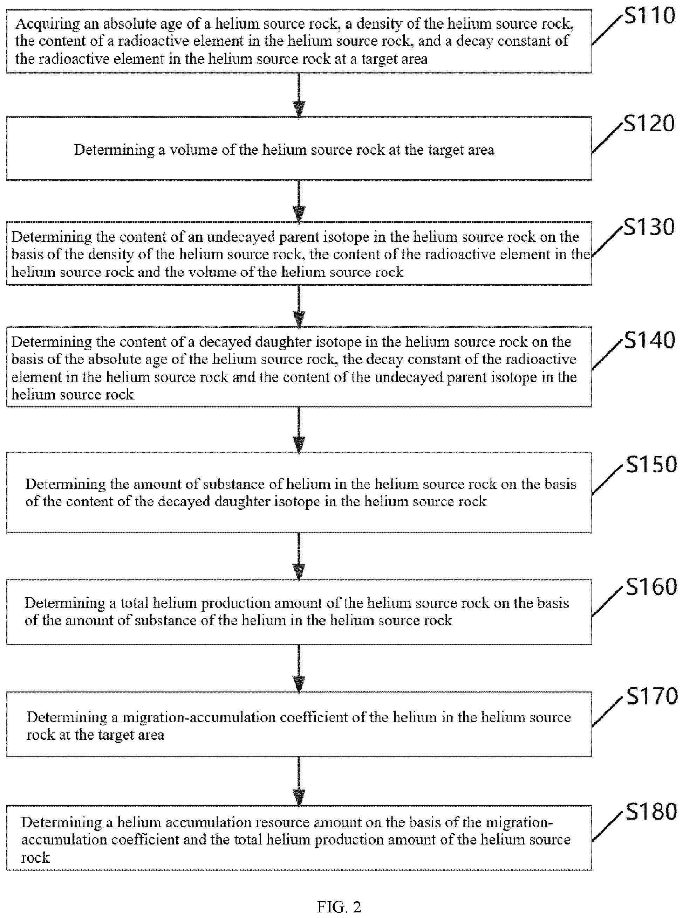

FIG. 2 schematically illustrates a flow diagram of a helium genesis resource evaluation method according to an embodiment of the present application. As shown in FIG. 2, in an embodiment of the present application, provided is a helium genesis resource evaluation method, this embodiment is mainly illustrated by taking the condition that the method is applied to the terminal 102 (or the server 104) in FIG. 1 as an example, and the method includes the following steps: Step 110, acquiring an absolute age of a helium source rock, a density of the helium source rock, the content of a radioactive element in the helium source rock, and a decay constant of the radioactive element in the helium source rock at a target area.

In this embodiment, the target area refers to a helium resource research area. In this embodiment, the helium source rock includes granite, a volcanic rock, a sedimentary rock, and the like. The age of the granite or the volcanic rock can be determined by measuring the isotopic age of zircon U-Pb in the rock. The age of the sedimentary rock is mainly determined by measuring the age of ancient extinct life in the rock. The density of the helium source rock is generally constant, e.g., the granite has a density of 2.3 $g/cm^3$. The radioactive element in helium source rock includes: uranium ($^{235}U$ and $^{238}U$) and thorium ($^{232}Th$). The content of radioactive elements such as uranium or thorium in the helium source rock can be determined by collecting representative helium source rock samples at different levels and structural parts. The content of uranium or thorium can be measured for multiple times with an error of less than 5% for the same sample, and is averaged. A decay constant of $^{235}$U is $9.8485\times10^{-10}$/a, a decay constant of $^{238}$U is $1.55125\times10^{-10}$/a, and a decay constant of $^{232}$Th is $4.9745\times10^{-11}$/a.

Step 120, determining a volume of the helium source rock at the target area.

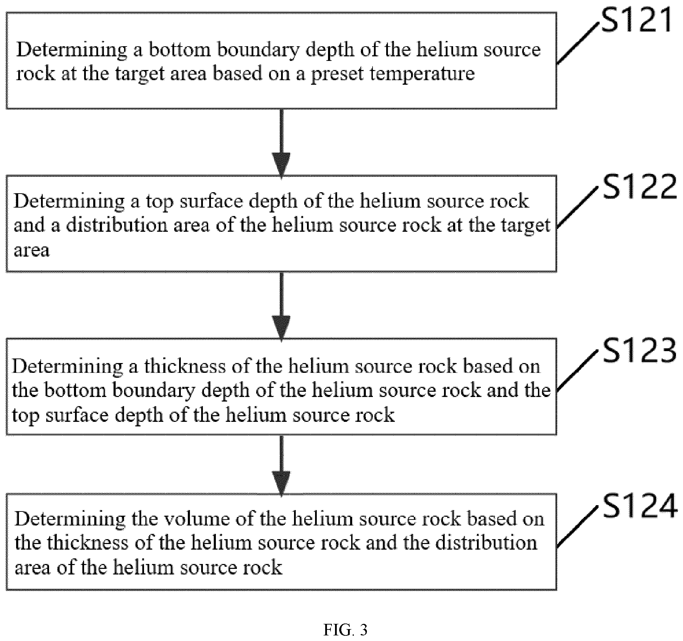

FIG. 3 schematically illustrates a flow chart of calculating a volume of a helium source rock at a target area in the helium genesis resource evaluation method according to the embodiment of the present application. As shown in FIG. 3, in this embodiment, the volume of the helium source rock at the target area is determined by Steps 121 to 124:

Step 121, determining a bottom boundary depth of the helium source rock at the target area based on a preset temperature.

Exemplarily, calculating the volume of the helium source rock first needs to calculate the bottom boundary depth of the helium source rock.

If the helium source rock is the granite or volcanic rock, the bottom boundary depth of the helium source rock may be determined from a formation depth corresponding to 220°C to 240°C.

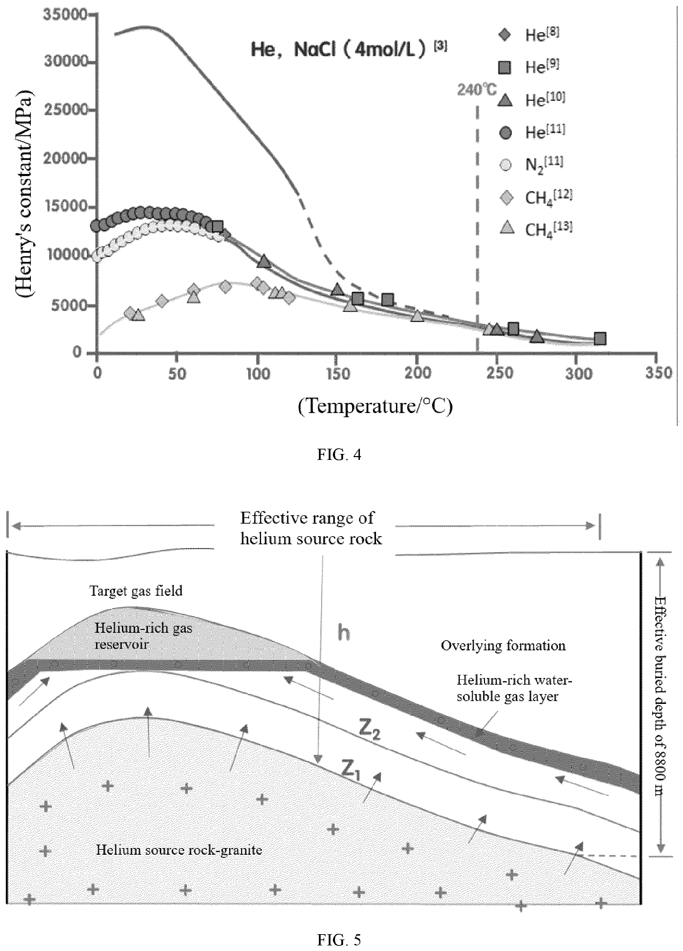

FIG. 4 schematically illustrates a curve graph of Henry's constants of He, $N_2$ and $CH_4$ as a function of temperature according to an embodiment of the present application. As shown in FIG. 4, according to the Henry curve, the solubility and partial pressure of helium (He) substantially tend to be consistent with those of methane ($CH_4$) and nitrogen ($N_2$) at 180°C, and the potential for further helium release substantially disappears, and the potential for helium release from the helium source rock tends to zero at 240°C. Therefore, in this embodiment, the formation depth corresponding to 220°C to 240°C in the research area is determined as a lower limit depth of the helium source rock such as the granite and the volcanic rock, i.e., the bottom boundary depth of the helium source rock.

FIG. 5 schematically illustrates an effective thickness of the helium source rock schematic according to an embodiment of the present application.

Exemplarily,, if the helium source rock is the granite or volcanic rock, the bottom boundary depth of the helium source rock may be calculated by a formula (1):

$$H_{bottom} = (t_{preset\ temperature}-t_{surface\ temperature})/K+50 \qquad (1);$$

wherein $H_{bottom}$ represents the bottom boundary depth of the helium source rock in m;
$t_{preset\ temperature}$ represents the preset temperature, and ranges from 220°C to 240°C;
$t_{surface\ temperature}$ represents the surface temperature in °C; and
K represents a geothermal gradient in °C/100 m.

If the helium source rock is the sedimentary rock, such as a coal seam or a mud shale, the bottom boundary depth of the helium source rock may be determined from seismic data and drilling data.

Step 122, determining a top surface depth of the helium source rock and a distribution area of the helium source rock at the target area.

In this embodiment, the top surface depth of the helium source rock and the distribution area of the helium source rock can be determined from the seismic data and the drilling data. For a helium source rock having an unstable thickness or a discontinuous distribution, the distribution area of the helium source rock can be found by a facet integration method.

Step 123, determining a thickness of the helium source rock based on the bottom boundary depth of the helium source rock and the top surface depth of the helium source rock.

Exemplarily, the thickness of the helium source rock may be calculated by a formula (2):

$$h = H_{bottom}-H_{top} \qquad (2);$$

wherein h represents the thickness of the helium source rock in m;
$H_{bottom}$ represents the bottom boundary depth of the helium source rock in m; and
$H_{top}$ represents the top surface depth of the helium source rock in m.

Step 124, determining the volume of the helium source rock based on the thickness of the helium source rock and the distribution area of the helium source rock.

Exemplarily, the volume of the helium source rock may be calculated by a formula (3):

$$V=h\times S \qquad (3);$$

wherein V represents the volume of the helium source rock in $m^3$;
h represents the thickness of the helium source rock in m; and
S represents the distribution area of the helium source rock in $m^2$.

This embodiment proposes a helium production method based on the Henry's law, solving a key difficulty in determining the thickness parameter of the helium source rock. A conventional method for determining a lower limit of the helium source rock adopts a subjective inference method to infer the thickness of an underground helium source rock according to the outcrop thickness of the helium source rock, but the thickness of the underground helium source rock varies greatly, resulting in inaccurate calculation of the volume of the helium source rock. In this application, guided by the theory of helium reservoir formation, a helium "death line" of 220°C-240°C is taken as the lower limit temperature for the depth of the helium source rock, and a depth corresponding to this temperature is taken as a lower limit of the depth of the helium source rock, so as to determine the thickness of the helium source rock, effectively improving the accuracy of the volume of the helium source rock and the helium production resource amount, with an error reduction of 10%. This embodiment effectively solves the problem existing in the prior art that the thickness of the helium source rock is difficult to determine.

Step 130, determining the content of an undecayed parent isotope in the helium source rock on the basis of the density of the helium source rock, the content of the radioactive element in the helium source rock and the volume of the helium source rock.

In this embodiment, the content of the undecayed parent isotope in the helium source rock can be calculated by a formula (4):

$$P = V \times \rho_{source\ rock} \times x \qquad (4);$$

wherein P represents the content of the undecayed parent isotope in the helium source rock in mol;
V represents the volume of the helium source rock in $m^3$;
$\rho_{source\ rock}$ represents the density of the helium source rock in $t/m^3$; and
x represents the content of the radioactive element in the helium source rock in mol/g.

Step 140, determining the content of a decayed daughter isotope in the helium source rock on the basis of the absolute age of the helium source rock, the decay constant of the radioactive element in the helium source rock and the content of the undecayed parent isotope in the helium source rock.

In this embodiment, the content of the decayed daughter isotope in the helium source rock can be calculated by a formula (5):

$$D = (e^{\lambda t}-1) \times V \times \rho_{source\ rock} \times x \qquad (5);$$

wherein D represents the content of the decayed daughter isotope in the helium source rock in mol;
$\lambda$ represents a decay constant of an isotope of the radioactive element in the helium source rock, dimensionless;
t represents an absolute geological age of the helium source rock in millions of years;
V represents the volume of the helium source rock in $m^3$;
$\rho_{source\ rock}$ represents the density of the helium source rock in $t/m^3$; and
x represents the content of the radioactive element in the helium source rock in mol/g.

Step 150, determining the amount of substance of helium in the helium source rock on the basis of the content of the decayed daughter isotope in the helium source rock.

In this embodiment, the radioactive element in the helium source rock includes: 238U and 232Th;

for decay of 238U, the amount of substance of the helium in the helium source rock is calculated by a formula (6):

$$n = D \times 8/238 \qquad (6);$$

for decay of 232Th, the amount of substance of the helium in the helium source rock is calculated by a formula (7):

$$n = D \times 6/232 \qquad (7);$$

wherein n represents the amount of substance of the helium in the helium source rock, and D represents the content of the decayed daughter isotope in the helium source rock.

Step 160, determining a total helium production amount of the helium source rock on the basis of the amount of substance of the helium in the helium source rock.

In this embodiment, the total helium production amount of the helium source rock is calculated a formula (8):

$$V_{He\ production} = n \times M/\rho_{he} \qquad\qquad (8);$$

wherein $V_{He\ production}$ represents the total helium production amount, i.e., a helium production resource amount in $m^3$; n represents the amount of substance of the helium in the helium source rock;
M represents a molar mass of the total helium production amount; and
$\rho_{he}$ represents the density of helium and is 0.1786 g/L.

Exemplarily, for the decay of 238U, the total helium production amount of the helium source rock is calculated by a formula (8.1):

$$V_{He\ production} = n \times M/\rho_{he}$$

$$= D \times M \times 8/238/\rho_{he}$$

$$= (e^{\lambda t}-1) \times v \times \rho_{source\ rock} \times x \times M \times 8/238/\rho_{he}\ (8.1);$$

Exemplarily, for the decay of 232Th, the total helium production amount of the helium source rock is calculated by a formula (8.2):

$$V_{He\ production} = n \times M/\rho_{he}$$

$$= D \times M \times 6/232/\rho_{he}$$

$$= (e^{\lambda t}-1) \times v \times \rho_{source\ rock} \times x \times M \times 6/232/\rho_{he}\ (8.2);$$

in the formulas (8.1)-(8.2), $V_{He\ production}$ represents the helium production resource amount in the research area, i.e., the total helium production amount of the helium source rock in $m^3$;
$\lambda$ represents a decay constant of an uranium or thorium isotope in the research area, dimensionless;
t represents the absolute geological age of the helium source rock in the research area in millions of years;
$\rho_{source\ rock}$ represents the density of the helium source rock in the research area in $t/m^3$; for example, the granite has a density of 2.5 $t/m^3$;
$\rho_{he}$ represents the density of helium in the research area and is 0.1786 g/L;
v represents the volume of the helium source rock in the research area in $m^3$; and
x represents the content of uranium or thorium in the helium source rock of the research area in g/mol.

Step 170, determining a migration-accumulation coefficient of the helium in the helium source rock at the target area.

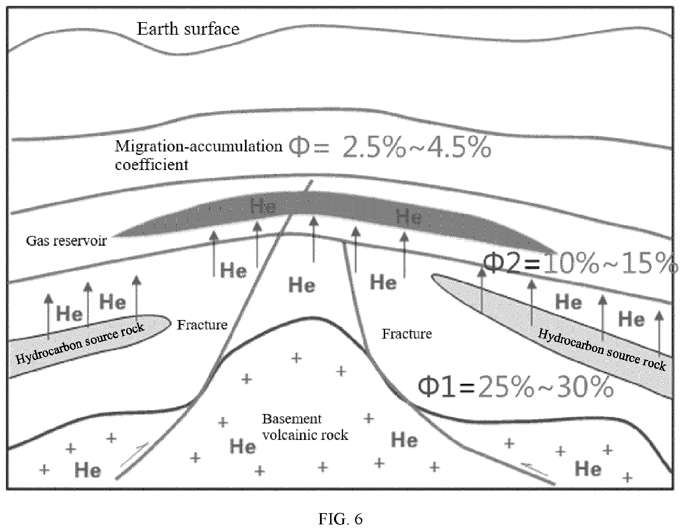

FIG. 6 schematically illustrates a helium reservoir formation mode and process according to an embodiment of the present application. As shown in FIG. 6, in this embodiment, a helium discharge rate of the helium source rock and a degassing rate of water-solution helium in the different reservoir formation modes are determined based on the structural activity phases and helium discharge characteristics in the research area, and the migration-accumulation coefficient can be obtained by multiplying the helium discharge rate of the helium source rock by the degassing rate of water-soluble helium. The different reservoir formation modes refer to accumulation forms of helium from different sources in different traps. According to the gas composition, helium reservoirs are divided into a helium-containing nitrogen reservoir, a helium-containing $CO_2$ reservoir, a helium-containing natural gas reservoir, etc.; according to the trap type, the traps are divided into an anticlinal trap, a fault trap, and a lithologic trap; and according to the source, helium is divided into mantle source helium, crust source helium, or mantle- and crust source helium, and the like.

A ratio of a helium source rock from which helium has been discharged to the volume of the total helium source rock is the helium discharge rate of the helium source rock. By measuring the content of water-soluble helium known in a scale area and the helium content in a gas reservoir, the amount of accumulated helium and the volume of water-soluble helium are calculated, and a ratio of the amount of accumulated helium to the volume of water-soluble helium is the degassing rate of water-soluble helium.

In this embodiment, the migration-accumulation coefficient of the helium in the helium source rock at the target area is calculated by a formula (9):

$$\Phi = \Phi 1 \times \Phi 2 \qquad (9);$$

wherein $\Phi 1$ is the helium discharge rate of the helium source rock in %; and $\Phi 2$ is the degassing rate of water-soluble helium in %.

The helium discharge rate $\Phi 1$ of the helium source rock is calculated by the ratio of the helium source rock from which helium has been discharged to the volume of the total helium source rock. By measuring the content of water-soluble helium known in the scale area and the helium content in the gas reservoir, the amount of accumulated helium and the volume of water-soluble helium are calculated, and a ratio of the amount of accumulated helium to the volume of water-soluble helium is the degassing rate $\Phi 2$ of water-soluble helium.

The primary and secondary migration-accumulation coefficients of helium vary greatly under different reservoir formation modes. The existing methods do not take into account the differences in the different reservoir formation modes, nor do they give specific calculation formulas. In this embodiment, the migration-accumulation coefficient is matched with the hydrocarbon reservoir formation time, solving the problem that the migration-accumulation coefficient is difficult to determine.

Step 180, determining a helium accumulation resource amount on the basis of the migration-accumulation coefficient and the total helium production amount of the helium source rock.

In this embodiment, the amount of helium geological resources in the research area is obtained by multiplying the total helium production amount of the granite, an igneous rock, the sedimentary rock and the like in the research area by the migration-accumulation coefficient.

In this embodiment, the helium accumulation resource amount is calculated by a formula (10):

$$V_{he\ accumulation} = \Phi \times V_{he\ production} \qquad (10);$$

wherein $V_{he\ accumulation}$ represents a helium accumulation amount, i.e., the helium accumulation resource amount in $m^3$;

$\Phi$ represents the migration-accumulation coefficient in %; and

$V_{he\ production}$ represents the total helium production amount in $m^3$.

In this embodiment, the helium includes $^3He$ and $^4He$, and the method further includes:

determining standard values for 3He/4He in crust source helium and mantle source helium, the standard values being as follows:

Value standard for crust source helium: $^3He/^4He=2.0\times10^{-8}$;

Value standard for mantle source helium: $^3He/^4He=1.1\times10^{-8}$; and

Value standard for crust and mantle source helium: $1.1\times10^{-8}<^3He/^4He<2.0\times10^{-8}$.

In this embodiment, the helium includes $^3He$ and $^4He$, and the method further includes calculating resources of mantle source helium and crust source helium by the ratio of $^3He/^4He$ according to a binary composite formula, and a ratio of the crust source helium is calculated as follows:

$$I_{3He} = \frac{R-R_c}{R_m-R_c} \qquad (11);$$

a ratio of mantle source helium resources is calculated as follows:

$$I_{4He} = 1 - I_{3He} \qquad (12);$$

wherein $^3He$ represents a proportion of the mantle source helium in the helium in %L;

$^4He$ represents a proportion of the crust source helium in the helium in %L;

R represents a measured volume of helium in $m^3$;

$R_c$ represents the volume of the crust source helium in $m^3$;

$R_m$ represents the volume of the mantle source helium in $m^3$.

FIG. 2 is a schematic flow diagram of a helium genesis resource evaluation method according to one embodiment. It should be understood that although the steps in the flow diagram of FIG. 2 are shown in sequence as indicated by the arrows, the steps are not necessarily performed in sequence in the order indicated by the arrows. The steps are performed without a strict order limitation and the steps may be performed in other orders unless explicitly stated herein. Moreover, at least some of the steps in FIG. 2 may include a plurality of sub-steps or a plurality of stages, which are not necessarily completed at the same time, but may be performed at different times, and the order of execution of these sub-steps or stages is not necessarily sequential, but these sub-steps or stages may be performed in turn or alternately with other steps or at least part of the sub-steps or stages of other steps.

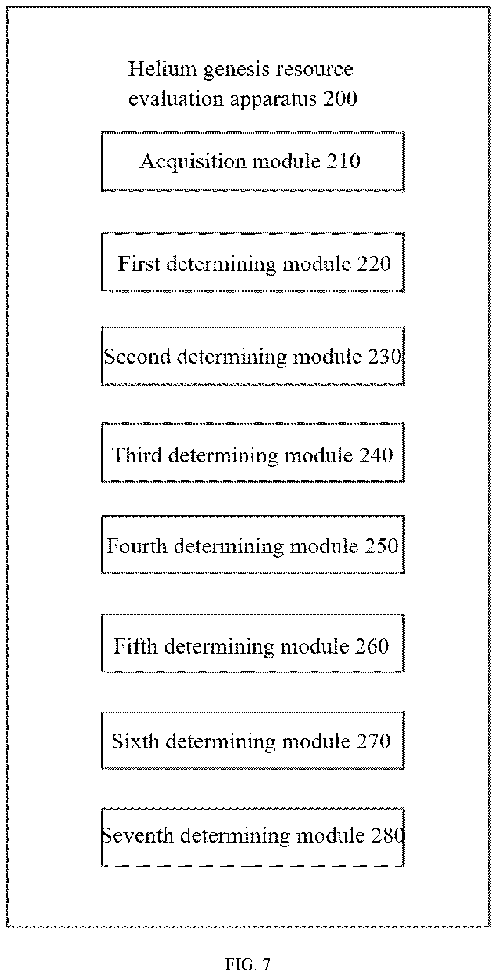

FIG. 7 is a structural block diagram schematically illustrating a helium genesis resource evaluation apparatus according to an embodiment of the present application.

In one embodiment, as shown in FIG. 7, provided is a helium genesis resource evaluation apparatus 200, including an acquisition module 210, a first determining module 220, a second determining module 230, a third determining module 240, a fourth determining module 250, a fifth determining module 260, a sixth determining module 270, and a seventh determining module 280, wherein:

the acquisition module 210 is configured to acquire an absolute age of a helium source rock, a density of the helium source rock, the content of a radioactive element in the helium source rock, and a decay constant of the radioactive element in the helium source rock at a target area;
the first determining module 220 is configured to determine a volume of the helium source rock at the target area;
the second determining module 230 is configured to determine the content of an undecayed parent isotope in the helium source rock on the basis of the density of the helium source rock, the content of the radioactive element in the helium source rock and the volume of the helium source rock;
the third determining module 240 is configured to determine the content of a decayed daughter isotope in the helium source rock on the basis of the absolute age of the helium source rock, the decay constant of the radioactive element in the helium source rock and the content of the undecayed parent isotope in the helium source rock;
the fourth determining module 250 is configured to determine the amount of substance of helium in the helium source rock on the basis of the content of the decayed daughter isotope in the helium source rock;
the fifth determining module 260 is configured to determine a total helium production amount of the helium source rock on the basis of the amount of substance of the helium in the helium source rock;
the sixth determining module 270 configured to determine a migration-accumulation coefficient of the helium in the helium source rock at the target area; and
the seventh determining module 280 is configured to determine a helium accumulation resource amount on the basis of the migration-accumulation coefficient and the total helium production amount of the helium source rock.

The first determining module 220 includes:

a bottom boundary depth determining module configured to determine a bottom boundary depth of the helium source rock at the target area based on a preset temperature;
a top surface depth and distribution area determining module configured to determine a top surface depth of the helium source rock and a distribution area of the helium source rock at the target area;
a thickness determining module configured to determine a thickness of the helium source rock based on the bottom boundary depth of the helium source rock and the top surface depth of the helium source rock; and
a volume determining module configured to determine the volume of the helium source rock based on the thickness of the helium source rock and the distribution area of the helium source rock.

The helium genesis resource evaluation apparatus includes a processor and a memory, the acquisition module 210, the first determining module 220, the second determining module 230, the third determining module 240, the fourth determining module 250, the fifth determining module 260, the sixth determining module 270 and the seventh determining module 280 and so on are each stored in the memory as a program unit, and the above program modules stored in the memory are executed by the processor to implement corresponding functions.

The processor includes a kernel that retrieves a corresponding program unit from the memory. One or more kernels may be provided, and the helium genesis resource evaluation method can be realized by adjusting kernel parameters.

The memory may include forms of a volatile memory, a random access memory (RAM) and/or a non-volatile memory in computer readable media, such as a read only memory (ROM) or a flash RAM, the memory including at least one memory chip.

An embodiment of the present application provides a storage medium having stored thereon a program which, when executed by a processor, implements the helium genesis resource evaluation method described above.

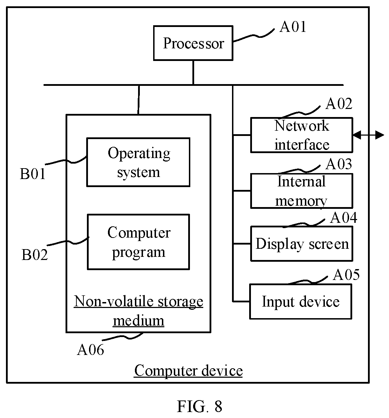

FIG. 8 schematically illustrates an internal structure of a computer device according to an embodiment of the present application.

In one embodiment, provided is a computer device, which may be a terminal, and an internal structure of the computer device may be shown in FIG. 8. The computer device includes a processor A01, a network interface A02, a display screen A04, an input device A05 and a memory (not shown in the figure) which are connected via a system bus. The processor A01 of the computer device is configured to provide computing and control capabilities. The memory of the computer device includes an internal memory A03 and a non-volatile storage medium A06. The non-volatile storage medium A06 stores an operating system B01 and a computer program B02. This internal memory A03 provides an environment for the operation of the operating system B01 and the computer program B02 in the non-volatile storage medium A06. The network interface A02 of the computer device is configured to communicate with an external terminal via a network connection. The computer program, when executed by the processor A01, implements the helium genesis resource evaluation method. The display screen A04 of the computer device may be a liquid crystal display screen or an electronic ink display screen, and the input device A05 of the computer device may be a touch layer covering the display screen, a key, a trackball or a trackpad disposed on a housing of the computer device, an external keyboard, trackpad or a mouse, or the like.

Those skilled in the art can understand that the structure shown in FIG. 8 merely shows a block diagram of a portion of the structures relevant to the solution of the present application, and does not constitute a limitation of a computer device to which the solution of the present application is applied, and a particular computer device may include more or fewer components than those shown in the figure, or combine some components, or have a different arrangement of components.

In one embodiment, the helium genesis resource evaluation apparatus provided in the present application may be implemented in the form of a computer program that can run on the computer device as shown in FIG. 8. The memory of the computer device may store various program modules constituting the helium genesis resource evaluation apparatus, such as the acquisition module 210, the first determining module 220, the second determining module 230, the third determining module 240, the fourth determining module 250, the fifth determining module 260, the sixth determining module 270, and the seventh determining module 280 shown in FIG. 7. The computer program constituted by the program modules causes a processor to execute the steps of the helium genesis resource evaluation method in the embodiments of the present application described in this specification.

The computer device shown in FIG. 8 may perform Step 110 by the acquisition module 210 in the helium cause resource evaluation apparatus as shown in FIG. 7, the computer device may perform Step 120 by the first determining module 220, the computer device may perform Step 130 by the second determining module 230, the computer device may perform Step 140 by the third determining module 240, the computer device may perform Step 150 by the fourth determining module 250, the computer device may perform Step 160 by the fifth determining module 260, the computer device may perform Step 170 by the sixth determining module 270, and the computer device may perform Step 180 by the seventh determining module 280.

An embodiment of the present application provides a device, including a processor, a memory, and a program stored on the memory and executable on the processor, the processor performing the following steps when executing the program:

Step 110, acquiring an absolute age of a helium source rock, a density of the helium source rock, the content of a radioactive element in the helium source rock, and a decay constant of the radioactive element in the helium source rock at a target area;

Step 120, determining a volume of the helium source rock at the target area;

Step 130, determining the content of an undecayed parent isotope in the helium source rock on the basis of the density of the helium source rock, the content of the radioactive element in the helium source rock and the volume of the helium source rock;

Step 140, determining the content of a decayed daughter isotope in the helium source rock on the basis of the absolute age of the helium source rock, the decay constant of the radioactive element in the helium source rock and the content of the undecayed parent isotope in the helium source rock;

Step 150, determining the amount of substance of helium in the helium source rock on the basis of the content of the decayed daughter isotope in the helium source rock;

Step 160, determining a total helium production amount of the helium source rock on the basis of the amount of substance of the helium in the helium source rock;

Step 170, determining a migration-accumulation coefficient of the helium in the helium source rock at the target area; and

Step 180, determining a helium accumulation resource amount on the basis of the migration-accumulation coefficient and the total helium production amount of the helium source rock.

In one embodiment, the Step 120 includes:

Step 121, determining a bottom boundary depth of the helium source rock at the target area based on a preset temperature;

Step 122, determining a top surface depth of the helium source rock and a distribution area of the helium source rock at the target area;

Step 123, determining a thickness of the helium source rock based on the bottom boundary depth of the helium source rock and the top surface depth of the helium source rock; and

Step 124, determining the volume of the helium source rock based on the thickness of the helium source rock and the distribution area of the helium source rock.

In one embodiment, the step that determining the bottom boundary depth of the helium source rock at the target area includes:

determining the bottom boundary depth of the helium source rock at the target area based on the preset temperature.

In one embodiment, determining the bottom boundary depth of the helium source rock at the target area based on the preset temperature includes:

determining a formation depth corresponding to the preset temperature as the bottom boundary depth of the helium source rock, the helium source rock including: granite and a volcanic rock.

In one embodiment, the preset temperature ranges from 220°C to 240°C.

In one embodiment, determining the bottom boundary depth of the helium source rock, the top surface depth of the helium source rock, and the distribution area of the helium source rock at the target area includes:

determining the bottom boundary depth of the helium source rock, the top surface depth of the helium source rock, and the distribution area of the helium source rock at the target area based on seismic data and drilling data, the helium source rock including: a coal seam and a mud shale.

In one embodiment, determining the distribution area of the helium source rock based on the seismic data and the drilling data includes:

obtaining the distribution area of the helium source rock by a facet integration method based on the seismic data and the drilling data for a helium source rock having an unstable thickness or a discontinuous distribution.

In one embodiment, the formation depth corresponding to the preset temperature is determined as the bottom boundary depth of the helium source rock by a formula (1):

$$H_{bottom} = (t_{preset\ temperature} - t_{surface\ temperature})/K + 50 \qquad (1);$$

wherein $H_{bottom}$ represents the bottom boundary depth of the helium source rock in m;

$t_{surface\ temperature}$ represents the surface temperature in °C; and

K represents a geothermal gradient in °C/100 m.

In one embodiment, the thickness of the helium source rock is determined based on the bottom boundary depth of the helium source rock and the top surface depth of the helium source rock by a formula (2):

$$h = H_{bottom} - H_{top} \qquad (2);$$

wherein h represents the thickness of the helium source rock in m;

$H_{bottom}$ represents the bottom boundary depth of the helium source rock in m; and

$H_{top}$ represents the top surface depth of the helium source rock in m.

In one embodiment, the volume of the helium source rock is determined based on the thickness of the helium source rock and the distribution area of the helium source rock by a formula (3):

$$V = h \times S \qquad (3);$$

wherein V represents the volume of the helium source rock in $m^3$;

h represents the thickness of the helium source rock in m; and

S represents the distribution area of the helium source rock in $m^2$.

In one embodiment, the content of the undecayed parent isotope in the helium source rock is determined on the basis of the density of the helium source rock, the content of the radioactive element in the helium source rock and the volume of the helium source rock by a formula (4):

$$P = v \times \rho_{source\ rock} \times x \qquad (4);$$

wherein P represents the content of the undecayed parent isotope in the helium source rock in mol;
v represents the volume of the helium source rock in $m^3$;
$\rho_{source\ rock}$ represents the density of the helium source rock in $t/m^3$; and
x represents the content of the radioactive element in the helium source rock in mol/g.

In one embodiment, the content of the decayed daughter isotope in the helium source rock is determined on the basis of the absolute age of the helium source rock, the decay constant of the radioactive element in the helium source rock, and the content of the undecayed parent isotope in the helium source rock by a formula (5):

$$D = (e^{\lambda t} - 1) \times v \times \rho_{source\ rock} \times x \qquad (5);$$

wherein D represents the content of the decayed daughter isotope in the helium source rock in mol;
$\lambda$ represents a decay constant of an isotope of the radioactive element in the helium source rock, dimensionless;
t represents an absolute geological age of the helium source rock in millions of years;
v represents the volume of the helium source rock in $m^3$;
$\rho_{source\ rock}$ represents the density of the helium source rock in $t/m^3$; and
x represents the content of the radioactive element in the helium source rock in mol/g.

In one embodiment, the radioactive element in the helium source rock includes: 238U and 232Th.
For decay of 238U, the amount of substance of the helium in the helium source rock is determined on the basis of the content of the decayed daughter isotope in the helium source rock by a formula (6):

$$n = D \times 8/238 \qquad (6);$$

for decay of 232Th, the amount of substance of the helium in the helium source rock is determined on the basis of the content of the decayed daughter isotope in the helium source rock by a formula (7):

$$n = D \times 6/232 \qquad (7);$$

wherein n represents the amount of substance of the helium in the helium source rock, and D represents the content of the decayed daughter isotope in the helium source rock.

In one embodiment, the total helium production amount of the helium source rock is determined on the basis of the amount of substance of the helium in the helium source rock by a formula (8):

$$V_{He\ production} = n \times M/\rho_{he} \qquad (8);$$

wherein $V_{He\ production}$ represents the total helium production amount, i.e., a helium production resource amount in $m^3$;
n represents the amount of substance of the helium in the helium source rock;
M represents a molar mass of the total helium production amount; and
$\rho_{he}$ represents the density of helium and is 0.1786 g/L.

In one embodiment, the migration-accumulation coefficient of the helium in the helium source rock at the target area is determined by a formula (9):

$$\Phi = \Phi 1 \times \Phi 2 \qquad (9);$$

wherein $\Phi 1$ is a helium discharge rate of the helium source rock in %; and $\Phi 2$ is a degassing rate of water-soluble helium in %.
In one embodiment, the helium accumulation resource amount is determined on the basis of the migration-accumulation coefficient and the total helium production amount of the helium source rock by a formula (10):

$$V_{he\ accumulation} = \Phi \times V_{he\ production} \qquad (10);$$

wherein $V_{he\ accumulation}$ represents a helium accumulation amount, i.e., the helium accumulation resource amount in $m^3$;

$\Phi$ represents the migration-accumulation coefficient in %; and

$V_{he\ production}$ represents the total helium production amount in $m^3$.

In one embodiment, the helium includes 3He and 4He, and the method further includes: determining resources of mantle source helium and crust source helium by a ratio of 3He/4He according to a binary composite formula.

It will be appreciated by those skilled in the art that the embodiments of the present application may be provided as a method, a system, or a computer program product. Accordingly, the present application may take the form of an entirely hardware embodiment, an entirely software embodiment or an embodiment combining software and hardware aspects. Furthermore, the present application may take the form of a computer program product embodied on one or more computer usable storage media (including but not limited to a disk storage, CD-ROM, an optical storage, etc.) having computer usable program codes embodied therein.

The present application is described with reference to flowcharts and/or block diagrams of methods, devices (systems), and computer program products according to the embodiments of the application. It should be understood that each flow and/or block in the flowcharts and/or block diagrams, and combinations of flows and/or blocks in the flowcharts and/or block diagrams can be implemented by computer program instructions. These computer program instructions may be provided to a processor of a general purpose computer, a special purpose computer, an embedded processor, or other programmable data processing devices to produce a machine, so that the instructions, which are executed by the processor of the computers or other programmable data processing devices, produce a device for implementing the functions specified in one or more flows of the flowcharts and/or one or more blocks of the block diagrams.

These computer program instructions may also be stored in a computer-readable memory that can direct a computer or other programmable data processing devices to function in a particular manner, so that instructions stored in the computer-readable memory are caused to produce an article of manufacture including an instruction device which implements the functions specified in one or more flows of the flowcharts and/or one or more blocks of the block diagrams.

These computer program instructions may also be loaded onto a computer or other programmable data processing devices, so that a series of operational steps are performed on the computer or other programmable devices to produce a computer-implemented process. Therefore, the instructions executed on the computer or other programmable devices provide steps for implementing the functions specified in one or more flows of the flowcharts and/or one or more blocks of the block diagrams.

In one typical configuration, a computing device includes one or more processors (CPUs), an input/output interface, a network interface, and a memory.

The memory may include forms of a volatile memory, a random access memory (RAM) and/or a non-volatile memory in a computer-readable medium, such as a read only memory (ROM) or a flash memory (flash RAM). The memory is an example of the computer-readable medium.

The computer-readable medium includes non-volatile and volatile, and removable and non-removable media that can realize information storage by any method or technology. The information may be computer readable instructions, data structures, modules of a program, or other data. Examples of a storage medium for a computer include but are not limited to a phase change random access memory (PRAM), a static random access memory (SRAM), a dynamic random access memory (DRAM), other types of random access memories (RAMs), a read-only memory (ROM), an electrically erasable programmable read-only memory (EEPROM), a flash memory or other memory technologies, a compact disc read-only memory (CD-ROM), a digital video disk (DVD) or other optical memories, magnetic cartridge tapes, magnetic tape storage, magnetic disk storage or other magnetic storage devices, or any other non-transmission medium, which can be configured to store information that can be accessed by a computing device. As defined herein, the computer-readable medium does not include transitory computer-readable media, such as modulated data signals and carrier waves.

It should also be noted that the term "include", "comprise" or any other variation thereof is intended to cover a non-exclusive inclusion, such that a process, method, article, or device that includes a list of elements includes not only those elements but also other elements not expressly listed or also includes elements inherent to such process, method, article, or device. Without more restrictions, the element defined by the statement "including one..." does not exclude that additional identical elements are still present in the process, method, article or device that include the elements.

The above are only embodiments of the present application, and are not intended to limit the present application. For those skilled in the art, various changes and variations can be made to the present application. Any modifications, equivalent replacements, improvements and the like made within the spirit and principle of the present application should be included within the scope of the claims of the present application.

**Claims**

1. A helium genesis resource evaluation method, comprising:

   acquiring an absolute age of a helium source rock, a density of the helium source rock, the content of a radioactive element in the helium source rock, and a decay constant of the radioactive element in the helium source rock at a target area;
   determining a volume of the helium source rock at the target area;
   determining the content of an undecayed parent isotope in the helium source rock on the basis of the density of the helium source rock, the content of the radioactive element in the helium source rock and the volume of the helium source rock;
   determining the content of a decayed daughter isotope in the helium source rock on the basis of the absolute age of the helium source rock, the decay constant of the radioactive element in the helium source rock and the content of the undecayed parent isotope in the helium source rock;
   determining the amount of substance of helium in the helium source rock on the basis of the content of the decayed daughter isotope in the helium source rock;
   determining a total helium production amount of the helium source rock on the basis of the amount of substance of the helium in the helium source rock;
   determining a migration-accumulation coefficient of the helium in the helium source rock at the target area; and
   determining a helium accumulation resource amount on the basis of the migration-accumulation coefficient and the total helium production amount of the helium source rock;
   wherein determining the volume of the helium source rock at the target area comprises:

      determining a bottom boundary depth of the helium source rock at the target area based on a preset temperature;
      determining a top surface depth of the helium source rock and a distribution area of the helium source rock at the target area;
      determining a thickness of the helium source rock based on the bottom boundary depth of the helium source rock and the top surface depth of the helium source rock; and
      determining the volume of the helium source rock based on the thickness of the helium source rock and the distribution area of the helium source rock.

2. The method according to claim 1, wherein determining the bottom boundary depth of the helium source rock at the target area based on the preset temperature comprises:

   determining a formation depth corresponding to the preset temperature as the bottom boundary depth of the helium source rock;
   wherein, the helium source rock comprising: granite and a volcanic rock.

3. The method according to claim 2, wherein the preset temperature ranges from 220°C to 240°C.

4. The method according to claim 1, wherein determining the top surface depth of the helium source rock and the distribution area of the helium source rock at the target area comprises:

   determining the top surface depth of the helium source rock and the distribution area of the helium source rock based on seismic data and drilling data;
   wherein, the helium source rock comprising: a coal seam and a mud shale;
   wherein, determining the distribution area of the helium source rock based on the seismic data and the drilling data comprises: obtaining the distribution area of the helium source rock by a facet integration method based on the seismic data and the drilling data for a helium source rock having an unstable thickness or a discontinuous distribution.

5. The method according to claim 3, wherein the formation depth corresponding to the preset temperature is determined as the bottom boundary depth of the helium source rock by a formula (1):

$$H_{bottom} = (t_{preset\ temperature} - t_{surface\ temperature})/K + 50 \qquad (1);$$

wherein $H_{bottom}$ represents the bottom boundary depth of the helium source rock in m;
$t_{surface\ temperature}$ represents the surface temperature in °C; and
K represents a geothermal gradient in °C/100 m.

6. The method according to claim 5, wherein the thickness of the helium source rock is determined based on the bottom boundary depth of the helium source rock and the top surface depth of the helium source rock by a formula (2):

$$h = H_{bottom}\text{-}H_{top} \qquad\qquad (2);$$

wherein h represents the thickness of the helium source rock in m;
$H_{bottom}$ represents the bottom boundary depth of the helium source rock in m; and
$H_{top}$ represents the top surface depth of the helium source rock in m.

7. The method according to claim 6, wherein the volume of the helium source rock is determined based on the thickness of the helium source rock and the distribution area of the helium source rock by a formula (3):

$$V = h \times S \qquad\qquad (3);$$

wherein V represents the volume of the helium source rock in $m^3$;
h represents the thickness of the helium source rock in m; and
S represents the distribution area of the helium source rock in $m^2$.

8. The method according to claim 7, wherein the content of the decayed daughter isotope in the helium source rock is determined on the basis of the absolute age of the helium source rock, the decay constant of the radioactive element in the helium source rock and the content of the undecayed parent isotope in the helium source rock by a formula (5):

$$D = (e^{\lambda t}\text{-}1) \times v \times \rho_{source\ rock} \times x \qquad\qquad (5);$$

wherein D represents the content of the decayed daughter isotope in the helium source rock in mol;
$v \times \rho_{source\ rock} \times x$ represents the content of the undecayed parent isotope in the helium source rock;
$\lambda$ represents a decay constant of an isotope of the radioactive element in the helium source rock, dimensionless;
t represents an absolute geological age of the helium source rock in millions of years;
v represents the volume of the helium source rock in $m^3$;
$\rho_{source\ rock}$ represents the density of the helium source rock in $t/m^3$; and
x represents the content of the radioactive element in the helium source rock in mol/g.

9. The method according to claim 8, wherein the radioactive element in the helium source rock comprises: 238U and 232Th;

for decay of 238U, the amount of substance of the helium in the helium source rock is determined on the basis of the content of the decayed daughter isotope in the helium source rock by a formula (6):

$$n = D \times 8/238 \qquad\qquad (6);$$

for decay of 232Th, the amount of substance of the helium in the helium source rock is determined on the basis of the content of the decayed daughter isotope in the helium source rock by a formula (7):

$$n = D \times 6/232 \qquad\qquad (7);$$

wherein n represents the amount of substance of the helium in the helium source rock, and D represents the content of the decayed daughter isotope in the helium source rock.

10. The method according to claim 9, wherein the total helium production amount of the helium source rock is determined on the basis of the amount of substance of the helium in the helium source rock by a formula (8):

$$V_{He\ production} = n \times M / \rho_{he} \qquad (8);$$

wherein $V_{He\ production}$ represents the total helium production amount, i.e., a helium production resource amount in $m^3$;

n represents the amount of substance of the helium in the helium source rock;

M represents a molar mass of the total helium production amount; and

$\rho_{he}$ represents the density of helium and is 0.1786 g/L.

11. The method according to claim 10, wherein the migration-accumulation coefficient of the helium in the helium source rock at the target area is determined by a formula (9):

$$\Phi = \Phi 1 \times \Phi 2 \qquad (9);$$

wherein $\Phi 1$ is a helium discharge rate of the helium source rock in %; and $\Phi 2$ is a degassing rate of water-soluble helium in %; and

the helium accumulation resource amount is determined on the basis of the migration-accumulation coefficient and the total helium production amount of the helium source rock by a formula (10):

$$V_{he\ accumulation} = \Phi \times V_{he\ production} \qquad (10);$$

wherein $V_{he\ accumulation}$ represents a helium accumulation amount, i.e., the helium accumulation resource amount in $m^3$;

$\Phi$ represents the migration-accumulation coefficient in %; and

$V_{he\ production}$ represents the total helium production amount in $m^3$.

12. The method according to claim 1, wherein the helium comprises 3He and 4He, and the method further comprises: determining resources of mantle source helium and crust source helium by a ratio of 3He/4He according to a binary composite formula.

13. A helium genesis resource evaluation apparatus, comprising:

an acquisition module configured to acquire an absolute age of a helium source rock, a density of the helium source rock, the content of a radioactive element in the helium source rock, and a decay constant of the radioactive element in the helium source rock at a target area;

a first determining module configured to determine a volume of the helium source rock at the target area;

a second determining module configured to determine the content of an undecayed parent isotope in the helium source rock on the basis of the density of the helium source rock, the content of the radioactive element in the helium source rock and the volume of the helium source rock;

a third determining module configured to determine the content of a decayed daughter isotope in the helium source rock on the basis of the absolute age of the helium source rock, the decay constant of the radioactive element in the helium source rock and the content of the undecayed parent isotope in the helium source rock;

a fourth determining module configured to determine the amount of substance of helium in the helium source rock on the basis of the content of the decayed daughter isotope in the helium source rock;

a fifth determining module configured to determine a total helium production amount of the helium source rock on the basis of the amount of substance of the helium in the helium source rock;

a sixth determining module configured to determine a migration-accumulation coefficient of the helium in the helium source rock at the target area; and

a seventh determining module configured to determine a helium accumulation resource amount on the basis of the migration-accumulation coefficient and the total helium production amount of the helium source rock;

wherein the first determining module comprises:

a bottom boundary depth determining module configured to determine a bottom boundary depth of the helium source rock at the target area based on a preset temperature;

a top surface depth and distribution area determining module configured to determine a top surface depth of the helium source rock and a distribution area of the helium source rock at the target area;

a thickness determining module configured to determine a thickness of the helium source rock based on the bottom boundary depth of the helium source rock and the top surface depth of the helium source rock; and a volume determining module configured to determine the volume of the helium source rock based on the thickness of the helium source rock and the distribution area of the helium source rock.

14. A processor, configured to perform the helium genesis resource evaluation method according to any one of claims 1-12.

15. A machine-readable storage medium, having stored thereon instructions, which, when executed by a processor, cause the processor to be configured to perform the helium genesis resource evaluation method according to any one of claims 1-12.

102

104

Network

FIG. 1

Acquiring an absolute age of a helium source rock, a density of the helium source rock, the content of a radioactive element in the helium source rock, and a decay constant of the radioactive element in the helium source rock at a target area /S110

Determining a volume of the helium source rock at the target area /S120

Determining the content of an undecayed parent isotope in the helium source rock on the basis of the density of the helium source rock, the content of the radioactive element in the helium source rock and the volume of the helium source rock /S130

Determining the content of a decayed daughter isotope in the helium source rock on the basis of the absolute age of the helium source rock, the decay constant of the radioactive element in the helium source rock and the content of the undecayed parent isotope in the helium source rock /S140

Determining the amount of substance of helium in the helium source rock on the basis of the content of the decayed daughter isotope in the helium source rock /S150

Determining a total helium production amount of the helium source rock on the basis of the amount of substance of the helium in the helium source rock /S160

Determining a migration-accumulation coefficient of the helium in the helium source rock at the target area /S170

Determining a helium accumulation resource amount on the basis of the migration-accumulation coefficient and the total helium production amount of the helium source rock /S180

FIG. 2

```
┌─────────────────────────────────────────────────┐   ╱S121
│ Determining a bottom boundary depth of the       │  ╱
│ helium source rock at the target area based on a  │
│ preset temperature                                │
└─────────────────────────────────────────────────┘
                        │
                        ▼
┌─────────────────────────────────────────────────┐   ╱S122
│ Determining a top surface depth of the helium     │  ╱
│ source rock and a distribution area of the helium │
│ source rock at the target area                    │
└─────────────────────────────────────────────────┘
                        │
                        ▼
┌─────────────────────────────────────────────────┐   ╱S123
│ Determining a thickness of the helium source rock │  ╱
│ based on the bottom boundary depth of the helium  │
│ source rock and the top surface depth of the      │
│ helium source rock                                │
└─────────────────────────────────────────────────┘
                        │
                        ▼
┌─────────────────────────────────────────────────┐   ╱S124
│ Determining the volume of the helium source rock  │  ╱
│ based on the thickness of the helium source rock  │
│ and the distribution area of the helium source    │
│ rock                                              │
└─────────────────────────────────────────────────┘
```

FIG. 3

FIG. 4

FIG. 5

FIG. 6

Helium genesis resource
evaluation apparatus 200

Acquisition module 210

First determining module 220

Second determining module 230

Third determining module 240

Fourth determining module 250

Fifth determining module 260

Sixth determining module 270

Seventh determining module 280

FIG. 7

A01

Processor

Network
interface — A02

Operating
system
B01

Internal
memory — A03

Computer
program
B02

Display screen — A04

Non-volatile storage
medium

Input device — A05

A06

Computer device

FIG. 8

# EP 4 597 174 A1

## INTERNATIONAL SEARCH REPORT

International application No.

**PCT/CN2022/138085**

### A. CLASSIFICATION OF SUBJECT MATTER

G01V11/00(2006.01)i

According to International Patent Classification (IPC) or to both national classification and IPC

### B. FIELDS SEARCHED

Minimum documentation searched (classification system followed by classification symbols)

IPC: G01V

Documentation searched other than minimum documentation to the extent that such documents are included in the fields searched

Electronic data base consulted during the international search (name of data base and, where practicable, search terms used)

CNABS, CNTXT, EPTXT, WOTXT, USTXT, CNKI, IEEE: 氦气, 评价, 氦源岩, 衰变, 同位素, 体积, helium, evaluation, helium source rock, decayed, isotopes, volume

### C. DOCUMENTS CONSIDERED TO BE RELEVANT

| Category* | Citation of document, with indication, where appropriate, of the relevant passages | Relevant to claim No. |
|---|---|---|
| A | CN 114910976 A (NORTHWEST INSTITUTE OF ECO-ENVIRONMENT AND RESOURCES, CHINESE ACADEMY OF SCIENCES) 16 August 2022 (2022-08-16) description, paragraphs [0043]-[0070] | 1-15 |
| A | CN 111308577 A (CHINA PETROLEUM & CHEMICAL CORP. et al.) 19 June 2020 (2020-06-19) entire document | 1-15 |
| A | CN 111338001 A (CHINA PETROLEUM & CHEMICAL CORP. et al.) 26 June 2020 (2020-06-26) entire document | 1-15 |
| A | US 2018202264 A1 (RS ENERGY GROUP TOPCO, INC.) 19 July 2018 (2018-07-19) entire document | 1-15 |
| A | US 4378055 A (PHILLIPS PETROLEUM COMPANY) 29 March 1983 (1983-03-29) entire document | 1-15 |

☐ Further documents are listed in the continuation of Box C. ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **06 June 2023** | **14 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2022/138085**

| Patent document cited in search report | | | Publication date (day/month/year) | Patent family member(s) | | | Publication date (day/month/year) |
|---|---|---|---|---|---|---|---|
| CN | 114910976 | A | 16 August 2022 | None | | | |
| CN | 111308577 | A | 19 June 2020 | None | | | |
| CN | 111338001 | A | 26 June 2020 | None | | | |
| US | 2018202264 | A1 | 19 July 2018 | WO | 2017139271 | A2 | 17 August 2017 |
| | | | | CA | 3001146 | A1 | 17 August 2017 |
| | | | | US | 10260319 | B2 | 16 April 2019 |
| | | | | EP | 3414428 | A2 | 19 December 2018 |
| US | 4378055 | A | 29 March 1983 | None | | | |

Form PCT/ISA/210 (patent family annex) (July 2022)